# EUROPÄISCHE PATENTSCHRIFT

(11) **EP 1 592 964 B1**
(45) Veröffentlichungstag und Bekanntmachung des Hinweises auf die Patenterteilung: **17.01.2007**
(21) Anmeldenummer: 04710845.1
(22) Anmeldetag: 13.02.2004
(51) Int. Cl.: G01N 22/00, G01N 22/04, G01N 33/36, D01G 31/00, D01H 13/32

(54) **MIKROWELLENRESONATOR UND TEXTILMASCHINE MIT EINEM DERARTIGEN RESONATOR**
MICROWAVE RESONATOR AND TEXTILE MACHINE COMPRISING SUCH A RESONATOR
RESONATEUR MICRO-ONDES ET MACHINE TEXTILE EQUIPEE D'UN RESONATEUR DE CE TYPE

(30) Priorität: 13.02.2003 DE 10306217
(43) Veröffentlichungstag der Anmeldung: 09.11.2005
(73) Patentinhaber: Rieter Ingolstadt Spinnereimaschinenbau AG, 85055 Ingolstadt (DE)
(72) Erfinder: KOVACS, Ottmar, 85055 Berching (DE); GÖHLER, Wolfgang, 85101 Lenting (DE); CHERIF, Chokri, 85057 Ingolstadt (DE)
(74) Vertreter: Schlief, Thomas P.
(86) Internationale Anmeldenummer: PCT/EP2004/001352
(87) Internationale Veröffentlichungsnummer: WO 2004/072630

(56) Entgegenhaltungen:
- EP-A- 0 967 479
- WO-A-00/12974
- DE-A- 4 004 119
- US-A- 5 103 180
- US-A- 5 369 368
- US-A- 5 977 780
- HOLLINGER RICHARD D ET AL: "Microwave characterization of dielectric materials from 8 to 110 GHz using a free-space setup" MICROWAVE OPT TECHNOL LETT;MICROWAVE AND OPTICAL TECHNOLOGY LETTERS 2000 JOHN WILEY & SONS INC, NEW YORK, NY, USA, [Online] Bd. 26, Nr. 2, 2000, Seiten 100-105, XP002278175 Gefunden im Internet: <URL:http://www.personal.psu.edu/faculty/j /a/jak29/Publications/Microwave%20characte rization%20of%20dielectric%20materials%20f rom%208%20to%20110%20GHz%20using%20a%20fre e-space%20setup.pdf> [gefunden am 2004-04-27]
- PATENT ABSTRACTS OF JAPAN vol. 1999, no. 04, 30. April 1999 (1999-04-30) & JP 11 023492 A (ATSUKUSU:KK;CORP MIYUKI:KK), 29. Januar 1999 (1999-01-29)

## Beschreibung

Die Erfindung betrifft einen Mikrowellenresonator zum Anschließen an eine Meßeinrichtung zur Messung der Banddicke und/oder der Feuchtigkeit von kontinuierlich durch den Resonatorraum gefördertem Fasermaterial sowie eine Textilmaschine mit einem derartigen Mikrowellenresonator.

Die Messung von Fasereigenschaften in der Textilindustrie ist unabdingbare Voraussetzung zur Produktion von hochwertigen Textilien. So ist beispielsweise die Messung von Faserbanddicken insbesondere zum Zwecke der Ausregulierung von Ungleichmäßigkeiten von einem oder mehreren einer Spinnereivorbereitungsmaschine vorgelegten Faserbändern unabdingbar. Gleichfalls ist zur Qualitätskontrolle des verstreckten Materials am Maschinenausgang eine derartige Messung wünschenswert. Meßwerte zur Faserbanddicke (es sind auch die Bezeichnungen Bandquerschnitt oder Bandmasse gebräuchlich) werden neben der genannten Qualitätskontrolle auch zum Abstellen der Maschine herangezogen, wenn vorgegebene Dickengrenzwerte überschritten werden und somit kein hochwertiges Produkt mehr erhalten wird.

Bisher werden überwiegend mechanisch abtastende Sensoren zur Ermittlung der Banddicke von dem bzw. den Faserbändern eingesetzt. Auch sind kapazitive Meßorgane bekannt. Eine neue Methode zur Faserbanddickenmessung stellt hingegen die Verwendung von Mikrowellen dar. Hierbei werden von einem Mikrowellengenerator erzeugte Mikrowellen, deren Frequenzen bevorzugt von einem Rechner innerhalb gewisser Grenzen verändert werden, in einen Resonatorraum eines Mikrowellenresonators eingekoppelt, durch welches auch das zu vermessende Fasermaterial kontinuierlich hindurchgeführt wird. Entsprechend der Faserart, der Banddichten bzw. Banddicken und Bandgeometrie sowie der Bandfeuchtigkeit tritt bei einer charakteristischen Mikrowellenfrequenz ein Resonanzsignal auf, welches nach Auskopplung von einem Rechner zur Ermittlung der Banddichte bzw. Banddicke und/oder der Bandfeuchtigkeit auswertbar ist. Eine derartige Methode für andere Anwendungszwecke ist beispielsweise in der EP 0468023 B1 beschrieben. Die Vorteile eines derartigen Meßverfahrens mittels Mikrowellen liegen insbesondere darin, daß ein hochpräzises, berührungsloses Abtasten des Fasermaterials möglich sind. Mechanische Beeinträchtigungen des Bandes sowie Meßungenauigkeiten aufgrund der Trägheit von mechanischen Meßelementen scheiden aus.

Es hat sich herausgestellt, daß diverse Probleme bezüglich des Zusammenspiels des Resonators und des hindurchgeführten Fasermaterials bestehen. So verändert sich beispielsweise bei der bei Entfernung des Fasermaterials aus dem Resonatorraum - z.B. bei auslaufender Maschine oder bei Entfernung eines Bandstaus - die Leerresonanzfrequenz mit der Zeit aufgrund eines Temperaturabfalls im Resonatorraum. Bei einem erneuten Einführen von Fasermaterial in den Resonator ist dieser daher verstimmt und muß neu eingestellt werden. Andere Probleme betreffen Schwankungen der Resonanzfrequenzen im Betrieb, die ebenfalls nicht von dem Fasermaterial herrühren.

US5369368 offenbart einen Hohlraumresonator zum Bestimmen des Wassergehalts einer Substanz, die durch ein den Hohlraum durchsetzendes Keramikrohr fliesst, das mit einem Teflonrohr ausgekleidet ist.

WO0012974 offenbart einen Hohlraumresonator zur Bestimmung der Feuchte und Dichte von kontinuierlich durch den Resonator in einem Teflonrohr gefördertem Fasermaterial.

Es ist Aufgabe der vorliegenden Erfindung, den Mikrowellenresonator zur Messung von Dicke bzw. Masse und/oder Feuchte von hindurchgefördertem Fasermaterial zu verbessern.

Diese Aufgabe wird bei einem Mikrowellenresonator der eingangs genannten Art durch die Merkmale des Anspruchs 1 gelöst. Weiterhin wird diese Aufgabe durch eine Textilmaschine mit einem derartigen Resonator gelöst.

Der Resonator weist mindestens ein elektrisch nicht leitendes Dielektrikum auf, dessen Dielektrizitätskonstante bei Temperaturschwankungen weitgehend unverändert bleibt. Fällt die Temperatur bei entleertem Resonatorraum ab, verändert sich die Leerresonanzfrequenz kaum, so daß bei erneutem Einführen von Fasermaterial keine Verstimmung des Resonators auftritt. Eine Neukalibrierung des Resonators erübrigt sich somit in aller Regel.

Bevorzugt liegt die Dielektrizitätskonstante des mindestens einen Dielektrikuma bei den maschinenüblichen Betriebsbedingungen unterhalb von 12.

Besonders vorteilhaft ist es zudem, wenn das Dielektrikum im wesentlichen keine Feuchte aufnimmt. Ein Zielwert liegt hier beispielsweise bei 0,1 %. Viele Materialien, insbesondere Kunststoffe, nehmen bei steigenden Temperaturen Wasser auf mit der Folge, daß sich die Resonanzfrequenz aufgrund dieser Feuchte meßverfälschend verändert. Durch eine geeignete Materialwahl kann dies verhindert werden.

Bevorzugt ist das Material mindestens eines Dielektrikums zudem abriebfest, um einem Verschleiß durch Faserkontakt entgegenzuwirken.

Weiterhin ist es vorteilhaft, wenn sich mindestens ein Dielektrikum bei Temperaturschwankungen im wesentlichen nicht verformt. Derartige Verformungen haben ebenfalls eine Resonanzfrequenzverstimmung zur Folge, die bei geeigneter Materialwahl unterdrückt wird. Materialien mit geringem Wärmeausdehnungskoeffizienten sind beispielsweise Stähle mit hohem Nickelgehalt, beispielsweise Ni 36-Stahl und hierbei beispielsweise Invar®-Stahl.

Bevorzugt wird für mindestens ein Dielektrikum als Werkstoff Keramik, Polykarbonat oder ein Verbundwerkstoff, insbesondere mit eingebetteter Keramik, verwendet. Als Verbundwerkstoff hat sich insbesondere TMM^{®} der amerikanischen Firma Rogers als geeignet erwiesen, das ein Hydrokarbon-Keramik-Verbundwerkstoff mit einer sehr guten Temperaturstabilität und insbesondere einer gegenüber Temperaturschwankungen sehr stabilen Dielektrizitätskonstanten ist.

Für die Resonatorwände wird vorzugsweise ein Material mit geringer Wärmeausdehnung verwendet, um Meßverfälschungen aufgrund von temperaturabhängigen Verformungen zu verhindern. Eine vorteilhafte diesbezügliche Ausgestaltung ist hierbei die Verwendung eines Stahls mit hohem Nickelanteil, beispielsweise NI 36-Stahl und hier z.B. Invar®-Stahl, der einen geeigneten, sehr geringen Wärmeausdehnungskoeffizienten besitzt. Da sich allerdings Mikrowellen-Resonanzfelder bei der Verwendung von Invar®-Stahl nur schlecht ausbilden, wird in einer bevorzugten Ausführungsform der Resonatorraum innenwandig mit einer leitfähigen Beschichtung versehen, die beispielsweise von einer sauerstoffarmen Kupfer-Beschichtung gebildet ist. Diese Kupfer-beschichtung wird im Rahmen dieser Erfindung vorteilhafterweise von dem mindestens einen Dielektrikum gemäß dieser Erfindung überdeckt. Dies kann bevorzugt dadurch realisiert werden, daß das mindestens eine Dielektrikum direkt auf der leitfähigen Beschichtung angeordnet ist.

Der erwähnte Keramikverbundstoff TMM^{®} oder ähnliche Werkstoffe haben den Vorteil, daß ihre Dielektrizitätskonstanten nicht nur im wesentlichen - zumindest im interessierenden Temperaturbereich von 20°C bis 60°C - nahezu konstant, sondern zudem auch relativ klein sind. Da die Mikrowellen-Signalgeschwindigkeit abhängig ist von der Dielektrizitätskonstanten, bedingt eine kleine Dielektrizitätskonstante auch eine kleine Signalgeschwindigkeit und damit eine größere Wellenlänge der Resonanzsignale. Daher können bei gleichbleibender Ortsauflösung der Signale die Abmessungen des Resonatorraums vorteilhafterweise kleiner gewählt werden, als wenn für das mindestens eine Dielektrikum ein Material mit größerer Dielektrizitätskonstante gewählt würde. Die gleichen Vorteile ergeben sich selbstverständlich bei Materialien mit ähnlichen Eigenschaften wie TMM^{®}.

Aufgrund des Führungsrohrs können Textilfasern nicht in den übrigen Teil des Resonatorraums eindringen, so daß eine andernfalls von Zeit zu Zeit notwendige Reinigung hinfällig wird. Diese Ausführungsform ist z.B. dann vorteilhaft, wenn lediglich ein einziges Faserband durch den Resonator geführt wird, da hier der runde Rohrquerschnitt dem runden Faserbandquerschnitt anpaßbar ist. Ein solcher Resonator kann beispielsweise am Ausgang eines Streckwerks eingesetzt werden oder am Einlauf einer Strecke, welche das Band von einer vorgeschalteten Karde erhält.

Das Führungsrohr ist in einer bevorzugten Variante zumindest abschnittsweise in Faserbandlaufrichtung konisch bzw. trichterförmig ausgebildet, um das Fasermaterial für ein nachfolgendes Kalanderwalzenpaar schon zu einem bestimmten Grad zu verdichten.

Zum Zweck einer einfacheren Einfädelung des Fasermaterials in den Resonator kann die Eintrittsöffnung des Führungsrohres erweitert und insbesondere konisch ausgebildet sein.

Da das Führungsrohr auswechselbar ausgestaltet ist, kann beispielsweise je nach Bandfeinheit ein passendes Führungsrohr mit modifiziertem Innendurchmesser gewählt werden. Zweckmäßigerweise ist die Auswertung der ausgekoppelten Mikrowellensignale auf das jeweils verwendete Führungsrohr abzustimmen. Ein derartiger Neuabgleich der Auswertungssoftware kann sich erübrigen, wenn die Massen der verschiedenen Führungsrohre im Bereich der Mikrowellenausbreitung im wesentlichen gleich groß gewählt werden. Dies bedeutet eine entsprechend geeignete Geometriewahl der Rohre.

Anspruchsgemäß sind mindestens zwei Rohre als erfindungsgemäße Dielektrika vorgesehen, wobei ein inneres in einem äußeren auswechselbar angeordnet, beispielsweise eingeschoben, ist. Das Fasermaterial wird hierbei im inneren Rohr geführt. Wie gesagt können verschiedene innere Führungsrohre mit verschiedenen Innendurchmessern wechselweise in das vorzugsweise selbe äußere Rohr eingesetzt werden. Das äußere Rohr dient vornehmlich zur Aufnahme des inneren Rohres und ist bevorzugt in geeigneter Weise am Resonator angeordnet, beispielsweise mit einer randseitigen Außenwulst eingehängt. Die Genauigkeit der Resonanzsignalauswertung für die verschiedenen inneren Rohre kann wie gesagt durch Neuabgleich der Auswertungssoftware und/oder durch gleiche Masse der inneren Rohre im Mikrowellenausbreitungsbereich gewährleistet werden.

Vorteilhafterweise ist das Führungsrohr an seiner stromabwärtigen Seite mit einem Bandtrichter gekoppelt bzw. koppelbar, so daß diese Einheit eine Doppelfunktion bei minimalem Platzaufwand einnehmen kann. Bei einer direkten Verbindung miteinander kann der Bandtrichter an das Rohrende beispielsweise aufgesteckt, mit diesem verschraubt oder anderweitig verbunden sein. Alternativ ist auch eine einstückige Ausbildung von Führungsrohr und Bandtrichter möglich. Der Bandtrichter legt das verstreckte Faserband bevorzugt einem Klemmspalt eines nachfolgenden Kalanderwalzenpaares vor, so daß die freie vom Faserband zurückgelegte Wegstrecke zwischen Bandtrichter und Kalanderwalzenpaar möglichst kurz ist.

Vorteilhafte Weiterbildungen sind durch die Merkmale der Unteransprüche gekennzeichnet.

Im folgenden wird die Erfindung anhand der Figuren näher erläutert. Es zeigen:
- Figur 1: einen nicht erfindungsgemäßen Mikrowellenresonator, geschnitten entlang I-I gemäß der Figur 2;
- Figur 1a: eine geschnittene Detailansicht der erfindungsgemäßen Ausführungsform zweier ineineinander geschobener, als Dielektrika ausgebildeter Rohre;
- Figur 2: den Mikrowellenresonator der Figur 1 von oben gesehen (Vliesführungsdüse entfernt);

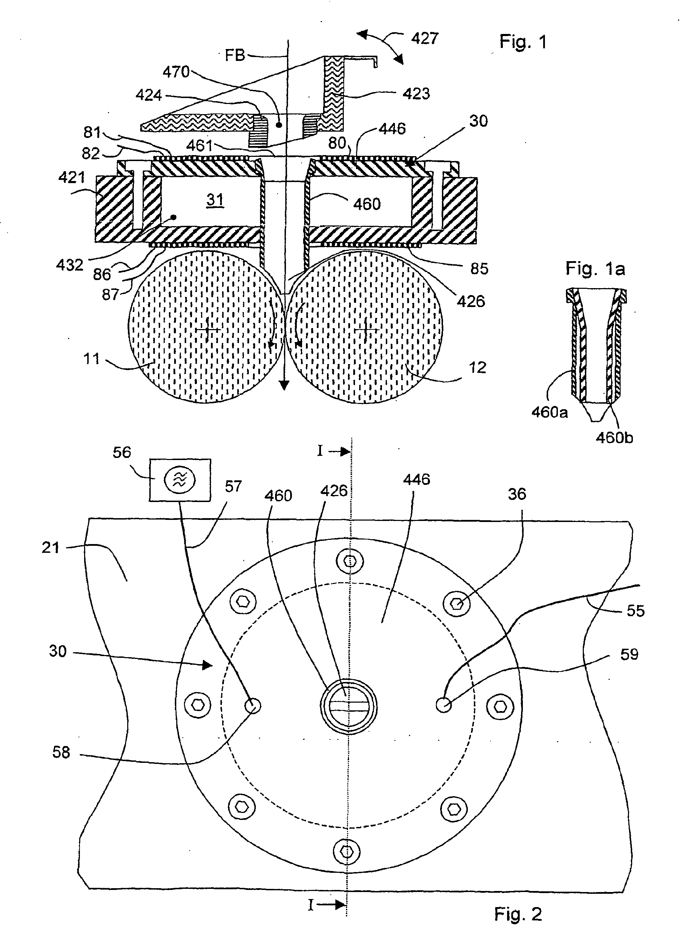

In den Figuren 1 und 2 ist eine nicht erfindungsgemäße Vorrichtung von der Seite im zentralen Schnitt und von oben dargestellt. Ein Resonator 30 ist in einer plattenförmigen Trägerkonstruktion 421 angeordnet. Die Trägerkonstruktion 421 weist hierzu eine zentrale Vertiefung 432 auf, die in der dargestellten Ausführungsform zylinderförmig ausgebildet ist, wie der Draufsicht der Figur 2 zu entnehmen ist. Auf die Vertiefung 432 ist ein Wandelement 446 aufgesetzt, das in der dargestellten Ausführungsform als flache Zylinderscheibe ausgebildet ist und randseitig Schraubenaufnahmen 36a aufweist, die mit entsprechenden Sackbohrungen 36b in der Trägerkonstruktion 421 fluchten. Wie in der Figur 2 dargestellt, können in diese Bohrungen 36a, 36b, welche jeweils Innengewinde aufweisen, Sechskantschrauben 36 eingeschraubt werden, um das Wandelement 446 mit der Trägerkonstruktion 21 zu verschrauben (die Schrauben sind in Figur 1 nicht dargestellt). In einer nicht dargestellten Alternative kann das Wandelement 446 in einer Ausnehmung in der Trägerkonstruktion 21 planparallel mit der Oberseite der Trägerkonstruktion 21 eingepaßt und verschraubt sein.

Das auf die Vertiefung 432 aufgesetzte Wandelement 446 läßt einen Resonatorraum 31 des Mikrowellenresonators 30 entstehen, in den Mikrowellen mit Hilfe eines Einkoppelelements 58 eingekoppelt und mit Hilfe eines Auskoppelelements 59 ausgekoppelt werden können, s. Figur 2. Beide beispielsweise stabförmig ausgebildeten Koppelelemente 58, 59 ragen durch entsprechende Bohrungen in dem Wandelement 46 von außen in den Resonatorraum 31. Das Einkoppelelement 58 ist über ein Kabel 57 an einen schematisch angedeuteten Mikrowellengenerator 56 angeschlossen, dessen Frequenz mit Hilfe einer nicht dargestellten Steuereinheit (vorzugsweise ein Mikroprozessor) variiert werden kann. Das Auskoppelelement 59 ist seinerseits über ein Kabel 55 mit einer nicht dargestellten Auswerteeinheit verbunden. Das Auskoppelelement 59 empfängt die im Resonator sich ausbildenden Mikrowellensignale und leitet sie an die Auswerteeinheit weiter, so daß diese zu aufeinander folgenden Zeitpunkten die jeweilige Resonanzfrequenz und die dazugehörige Signalbreite ermittelt werden kann. Aus diesen Informationen kann dann die Banddicke bzw. Bandmasse des jeweils gerade den Resonatorraum durchlaufenden Fasermaterials ermittelt werden.

In die Vertiefung 432 ist ein im wesentlichen als hohlzylinderförmiges Führungsrohr 460 ausgebildetes Dielektrikum 460 eingesetzt, das aus einem dielektrischen Material besteht. Die Durchgangsöffnung im Führungsrohr 460 fluchtet mit einer zentralen Durchgangsöffnung des Wandelements 446 sowie einer Durchgangsöffnung in der Trägerkonstruktion 421. Das nur schematisch als Pfeil dargestellte Faserband FB kann somit linear durch den Resonatorraum 31 direkt in den Klemmspalt zwischen zwei nachgeordneten Kalanderwalzen 11, 12 geführt werden.

Das Führungsrohr bzw. das Dielektrikum 460 ist aus einem Material gefertigt, dessen Dielektrizitätskonstante bei maschinenbetriebsüblichen Temperaturschwankungen - typischerweise zwischen 20°C und 60°C - im wesentlichen unverändert bleibt. Somit verändert sich die Leerresonanzfrequenz des Resonators 30 vom Zeitpunkt des Austritts eines Bandendes bis zum neuerlichen Einführen von Faserband - unabhängig von der dazwischen liegenden Zeitdauer - kaum, so daß keine neue Kalibrierung des Mikrowellensensors vorgenommen werden müßte.

Weiterhin ist das Material des Führungsrohr 460 vorteilhafterweise im wesentlichen abriebfest, nimmt im wesentlichen keine Feuchte auf und verformt sich weitgehend nicht bei Temperaturschwankungen.

Als Materialien, welche die oben genannten Anforderungen erfüllen, haben sich geeignete Keramiken, Polykarbonate, Hydrokarbon-Keramik-Verbundwerkstoffe und andere Verbundwerkstoffe erwiesen. Insbesondere erfüllt ein Verbundwerkstoff auf Kunststoffbasis mit eingebetteter Keramik mit dem Namen TMM^{®} diese Anforderungen.

Das Führungsrohr bzw. Dielektrikum 460 gemäß Figur 1 weist im Bereich des Wandelements 446 eine konische Erweiterung 461 auf, die ihrerseits eine Umfangswulst besitzt, die bei eingesetztem Führungsrohr 460 an einem entsprechend gestuften Rand der Durchgangsöffnung des Wandelements 446 zur Anlage kommt. Auf diese Weise ist einerseits eine sichere Lagerung des Führungsrohrs 460 im Resonator 30 sichergestellt, andererseits kann das Führungsrohr 460 schnell und problemlos gewechselt werden. Die gegenüberliegende Stirnseite des Führungsrohrs 460 ist als Bandtrichter 426 mit einem schnabelförmigen Endabschnitt ausgebildet. Der Bandtrichter 426 legt das Faserband FB möglichst nah in den Klemmspalt zwischen den Kalanderwalzen 11, 12 vor.

Das Führungsrohr 460 ist in einfacher Weise auszuwechseln, indem das Wandelement 446 losgeschraubt und abgenommen wird. Je nach Materialart und Verstreckungsbedingungen können verschiedene Einsätze 460 verwendet werden, die vorzugsweise dieselbe Masse im Mikrowellenausbreitungsbereich aufweisen, damit eine Neukalibrierung des Resonators nicht notwendig ist. Vorteilhafterweise ist hierbei der Außendurchmesser der jeweiligen Einsätze 460 bei unterschiedlichem innendurchmesser gleich.

Bei der einzigen erfindungsgemässen, in Figur 1a angedeuteten Ausführungsform sind mindestens zwei Rohre 460a, 460b als erfindungsgemäße Dielektrika vorgesehen, wobei ein inneres Rohr 460b in einem äußeren Rohr 460a angeordnet, beispielsweise eingeschoben, ist. Hierbei dient das innere Rohr 460b als Führungsrohr für das Faserband. Das äußere Rohr 460a dient vornehmlich zur Aufnahme des inneren Rohres 460b und ist bevorzugt in geeigneter Weise am Resonator angeordnet, beispielsweise mit seiner randseitigen Außenwulst eingehängt (wie das Führungsrohr 460 in Figur 1). Auf diese Weise können wahlweise verschiedene Führungsrohre 460b mit unterschiedlichem Innendurchmesser in vorzugsweise dasselbe äußere Rohr 460a eingesetzt werden, ohne das dieses entfernt werden müßte. Somit wird der Innenraum des Resonators auch beim Auswechseln der inneren Führungsrohre 460b vor Verschmutzung geschützt. Die Genauigkeit der Resonanzsignalauswertung für die verschiedenen inneren Rohre 460b kann bei dieser Ausführungsform ebenfalls durch Neuabgleich und/oder durch gleiche Massen der Dielektrika 460a, 460b erzielt werden.

Wie der Figur 1 zu entnehmen ist, ist oberhalb des Wandelements 446 eine Vliesführungsdüse 423 angeordnet, die eine Bohrung 470 aufweist, in die ein Vliesdüseneinsatz 424 eingesetzt und mittels einem nicht dargestellten Zentrierstift gehalten ist. Auf der dem Führungsrohr bzw. dem Dielektrikum 460 abgewandten Seite ist der Vliesdüseneinsatz 24 umfangseitig gerundet ausgebildet, um ein schonendes Einführen des Faserbandes FB in das Führungsrohr 460 zu gewährleisten. Die Vliesführungsdüse 423 ist derart an der Trägerkonstruktion angelenkt, daß sie in Richtung des Doppelpfeils 427 verschwenkbar ist, insbesondere im Falle eines Bandstaus an der Düse 423. Die Anlenkstellen der Vliesführungsdüse 423 an den Schmalseiten der Trägerkonstruktion 421 sind nicht dargestellt.

Auf der dem Resonator 30 abgewandten Seite des Wandelements 446 ist eine erste, elektrische Heizfolie 80 angebracht, während auf der gegenüberliegenden Seite der Trägerkonstruktion 421 außenseitig eine zweite Heizfolie 85 angeordnet ist. Beide Heizfolien 80, 85 sind über Anschlußdrähte 81, 82 bzw. 86, 87 mit einer nicht dargestellten Heizquelle verbunden. Die Heizleistung wird vorteilhafterweise geregelt, beispielsweise auf 35° C. Hierfür sind zweckmäßigerweise ein oder mehrere nicht dargestellte Temperaturmeßeinrichtungen vorgesehen, die beispielsweise in einer bzw. mehreren, nahe an den Resonatorraum 31 heranreichenden, seitlichen Bohrungen in der Trägerkonstruktion 421 angeordnet sein können. Eine thermische Hüllisolation, welche beispielsweise die gesamte Trägerkonstruktion - mit entsprechenden Öffnungen für das Fasermaterial - umgibt, kann zur Verhinderung des Einflusses von Temperaturschwankungen in der Umgebung sowie zur Verhinderung eines Heizleistungsverlustes gleichfalls vorgesehen sein.

Weitere zusätzliche oder alternative Maßnahmen zur Temperaturstabilisierung können darin bestehen, daß die den Resonatorraum 31 umgebenden Elemente aus einem Material mit geringer Wärmeausdehnung gefertigt sind, beispielsweise aus einem Stahl mit hohem Nickelanteil und hier z.B. aus Invar®-Stahl.

Die Innenwandung des Resonators 30 kann eine leitfähige Beschichtung aus beispielsweise sauerstoffarmem Kupfer aufweisen, da der Invar®-Stahl des Wandelements 446 und der Trägerkonstruktion 421 eine nur relativ geringe elektrische Leitfähigkeit besitzt. Mikrowellenresonanzen mit genügender Signalstärke könnten sich ohne eine solche leitfähige Beschichtung ggf. nicht ausbilden. Um eine Korrosion der Beschichtung zu verhindern, ist auf dieser zusätzlich eine korrosionsbeständige Beschichtung aus beispielsweise Gold oder Silber aufgebracht. Alternativ kann eine Keramik oder ein Verbundstoff mit eingebetteter Keramik als Beschichtung oder Abdeckung eingesetzt werden.

Der Resonator 30 mit dem als Führungsrohr 460 ausgebildeten Dielektrikum kann vorteilhafterweise hinter einem Streckwerk angeordnet werden. Das das Streckwerk verlassende Faservlies wird zu einem Band geformt wird und anschließend in den Resonator 30 eingeführt. In einer Alternative kann der Resonator 30 zwischen einer Karde und einer Strecke angeordnet sein, wobei das die Karde verlassende Fasermaterial ohne eine Zwischenablage in eine Kanne in das Streckwerk der Strecke transportiert wird.

Die beschriebenen Mikrowellenresonatoren mit Mikrowellengenerator können beispielsweise an einer Strecke mit einem regulierten oder unregulierten Streckwerk eingesetzt werden. Bei einem regulierten Streckwerk kann ein Mikrowellensensor vor und nach dem Streckwerk angeordnet sein. Die Erfindung läßt sich beispielsweise ebenfalls ohne Einschränkung bei einer Karde oder einer Kämmmaschine einsetzen.

## Patentansprüche

1. Mikrowellenresonator für eine oder an einer Textilmaschine, insbesondere Karde, Strecke oder Kämmmaschine, zum Anschließen an eine Meßeinrichtung zur Messung der Dicke und/oder der Feuchtigkeit von kontinuierlich durch den Resonatorraum (31) gefördertem Fasermaterial (FB), wobei im Resonator (30) zumindest bereichsweise mindestens ein elektrisch nicht leitendes Dielektrikum (460; 460a, 460b) vorgesehen ist, dessen Dielektrizitätskonstante bei maschinenbetriebsüblichen Temperaturschwankungen im wesentlichen unverändert bleibt, und wobei mindestens zwei als besagte Dielektrika ausgebildete, im Resonator (30) angeordnete und an beiden Stirnseiten offene Rohre (460a, 460b) vorgesehen sind, **dadurch gekennzeichnet, daß** ein inneres Führungsrohr (460b), das zur Hindurchführung des Fasermaterials (FB) ausgebildet ist; auswechselbar in ein äußeres Rohr (460a) eingesetzt ist.

2. Mikrowellenresonator nach Anspruch 1, **dadurch gekennzeichnet, daß** die Dielektrizitätskonstante der Dielektrika (460a, 460b) derart ist, daß sich die Leerresonanzfrequenz nach Entfernen des Fasermaterials (FB) aus dem Resonator (30) während des damit einhergehenden Temperaturabfalls nicht wesentlich verändert.

3. Mikrowellenresonator nach Anspruch 1, **dadurch gekennzeichnet, daß** die Dielektrizitätskonstante der Dielektrika (460a, 460b) bei den maschinenbetriebsüblichen Bedingungen unterhalb von 12 liegt.

4. Mikrowellenresonator nach einem der vorhergehenden Ansprüche, **dadurch gekennzeichnet, daß** die Dielektrika (460a, 460b) im wesentlichen keine Feuchte aufnehmen.

5. Mikrowellenresonator nach einem der vorhergehenden Ansprüche, **dadurch gekennzeichnet, daß** sich die Dielektrika (460a, 460b) bei Temperaturschwankungen im wesentlichen nicht verformen.

6. Mikrowellenresonator nach einem der vorhergehenden Ansprüche, **dadurch gekennzeichnet, daß** mindestens ein Dielektrikum (460a, 460b) aus Keramik, Polykarbonat, einem Hydrokarbon-Keramik-Verbundwerkstoff und/oder einem sonstigen Verbundwerkstoff, insbesondere mit eingebetteter Keramik, gefertigt ist.

7. Mikrowellenresonator nach einem der vorhergehenden Ansprüche, **dadurch gekennzeichnet, daß** mindestens ein Dielektrikum (460a, 460b) aus TMM® besteht.

8. Mikrowellenresonator nach einem der vorhergehenden Ansprüche, **dadurch gekennzeichnet, daß** das Führungsrohr (460b) zumindest abschnittsweise konisch ausgebildet ist.

9. Mikrowellenresonator nach einem der vorhergehenden Ansprüche, **dadurch gekennzeichnet, daß** das Führungsrohr (460b) eine erweiterte, vorteilhafterweise konisch ausgebildete, Eintrittsöffnung (461) aufweist.

10. Mikrowellenresonator nach einem der vorhergehenden Ansprüche, **dadurch gekennzeichnet, daß** im Bereich des stromabwärtigen Endes des Führungsrohrs (460b) ein mit der zugehörigen stimseitigen Rohröffnung fluchtender Bandtrichter (426) angeordnet oder anordenbar ist.

11. Mikrowellenresonator nach Anspruch 10, **dadurch gekennzeichnet, daß** das Führungsrohr (460b) und der Bandtrichter (426) einstückig ausgebildet sind.

12. Mikrowellenresonator nach einem der vorhergehenden Ansprüche, **dadurch gekennzeichnet, daß** verschiedene alternativ in den Resonator (30) einsetzbare Führungsrohre (460b) vorgesehen sind, die jeweils im wesentlichen den gleichen Außendurchmesser und voneinander verschiedene Innendurchmesser aufweisen.

13. Mikrowellenresonator nach einem der vorhergehenden Ansprüche, **dadurch gekennzeichnet, daß** verschiedene innere Führungsrohre (460b) mit unterschiedlichem Innendurchmesser in das äußere Rohr (460a) einsetzbar sind.

14. Mikrowellenresonator nach einem der vorhergehenden Ansprüche, **dadurch gekennzeichnet, daß** verschiedene, alternativ in den Resonator (30) einsetzbare Führungsrohre (460b) mit unterschiedlichem Innendurchmesser im wesentlichen die gleiche Masse im Bereich der Mikrowellenausbreitung aufweisen.

15. Textilmaschine, insbesondere Karde, Strecke oder Kämmmaschine, **dadurch gekennzeichnet, dass** sie mindestens einen Mikrowellenresonator (30) nach einem der vorhergehenden Ansprüche enthält.

## Claims

1. A microwave resonator for or on a textile machine, especially a machine for carding, drafting or combing, for connecting to a measurement apparatus for the measurement of the thickness and/or the water content of fiber material (FB), which is continually being transported through the resonator space (31), whereby, in the resonator (30), at least area-wise, at least one electrical non-conducting dielectric (460; 460a, 460b) is provided, the dielectric constant of which remains essentially unchanged at normal machine operational temperature variations, and whereby at least two tubes (460a, 460b) open at each end facing are provided and placed in the resonator (30), which serve as said dielectrics, **characterized in that** an inner guide tube (460b), which is designed for the throughput of fiber material (FB), is exchangeably inserted into an outer tube (460a).

2. A microwave resonator according to claim 1, **characterized in that** the dielectric constant of the dielectrics (460a, 460b) is of such a nature, that the empty resonance frequency does not essentially change itself after removal of fiber material (FB) from the resonator (30) and the therewith associated drop in temperature.

3. A microwave resonator according to claim 1, **characterized in that** the dielectric constant of the dielectrics (460a, 460b) stands at less than 12 during normal machine operating conditions.

4. A microwave resonator according to one of the foregoing claims, **characterized in that** the dielectrics (460a, 460b) essentially lack an affinity for moisture.

5. A microwave resonator according to one of the foregoing claims, **characterized in that** the dielectrics (460a, 460b) essentially are not deformed by temperature variations.

6. A microwave resonator according to one of the foregoing claims, **characterized in that** at least one dielectric (460a, 460b) is made of ceramics, polycarbonate, a hydrocarbon-ceramic composition material and/or other composition material, especially with embedded ceramics.

7. A microwave resonator according to one of the foregoing claims, **characterized in that** at least one dielectric (460a, 460b) consists of TMM®.

8. A microwave resonator according to one of the foregoing claims, **characterized in that** the guide tube (460b) is designed at least sectionally as having a conical shape.

9. A microwave resonator according to one of the foregoing claims, **characterized in that** the guide tube (460b) possesses a widened, advantageously conically tapered entry opening (461).

10. A microwave resonator according to one of the foregoing claims, **characterized in that** in the area of the downstream end of the guide tube (460b) is placed, or can be placed, a band funnel (426) in alignment with the thereto associated end face of the said guide tube.

11. A microwave resonator according to claim 10, **characterized in that** the guide tube (460b) and the band funnel (426) are of one-piece construction.

12. A microwave resonator according to one of the foregoing claims, **characterized in that** various guide tubes (460b) are provided, which can be alternately installed in the resonator (30) and which commonly possess, essentially, the same outside diameter, but respectively differ from one another in the inside diameters.

13. A microwave resonator according to one of the foregoing claims, **characterized in that** different inner guide tubes (460b) with different inside diameters can be inserted into the outer tube (460a).

14. A microwave resonator according to one of the foregoing claims, **characterized in that** different guide tubes (460b), which can be alternatively installed in the resonator (30) and are provided with different inside diameters, possess essentially the same mass in the area of microwave propagation.

15. A textile machine, especially card, drawing frame or combing machine, **characterized in that** it comprises at least one microwave resonator (30) according to one of the foregoing claims.

## Revendications

1. Résonateur à hyperfréquences pour une machine textile ou à une machine textile, particulièrement une cardeuse, une étireuse ou une peigneuse, pour le raccordement à une dispositif de mesure pour la mesure de l'épaisseur et/ou de l'humidité de matières fibreuses (FB) transportées en continu à travers l'espace de résonateur (31), sachant qu'il est prévu dans le résonateur (30) au moins en certaines parties au moins un diélectrique non conducteur électrique (460; 460a, 460b), dont la constante diélectrique reste essentiellement constante lors des fluctuations de température propres au fonctionnement habituel de la machine, et sachant que deux tubes (460a, 460b) au moins se présentant sous la forme desdits diélectriques, disposés dans le résonateur (30) et ouverts en leurs deux bouts sont prévus, **caractérisé en ce qu'**un tube de guidage intérieur (460b) servant au passage de la matière fibreuse (FB) est disposé de manière remplaçable dans un tube extérieur (460a).

2. Résonateur à hyperfréquences selon la revendication 1, **caractérisé en ce que** la constante diélectrique des diélectriques (460a, 460b) est telle que la fréquence de résonance à vide ne varie pas significativement après l'enlèvement de la matière fibreuse (FB) du résonateur (30) lors de la baisse de température qui accompagne ledit enlèvement.

3. Résonateur à hyperfréquences selon la revendication 1, **caractérisé en ce que** la constante diélectrique des diélectriques (460a, 460b) est inférieure à 12 dans les conditions de fonctionnement habituelles de la machine.

4. Résonateur à hyperfréquences selon l'une quelconque des revendications précédentes, **caractérisé en ce que** les diélectriques (460a, 460b) n'enregistrent pas essentiellement de l'humidité.

5. Résonateur à hyperfréquences selon l'une quelconque des revendications précédentes, **caractérisé en ce que** les diélectriques (460a, 460b) ne se déforment pas sensiblement lors des fluctuations de température.

6. Résonateur à hyperfréquences selon l'une quelconque des revendications précédentes, **caractérisé en ce qu'**un diélectrique (460a, 460b) au moins est réalisé en céramique, en polycarbonate, en un matériau composite d'hydrocarbone et de céramique et/ou d'un autre matériau composite quelconque, particulièrement avec céramique enrobée.

7. Résonateur à hyperfréquences selon l'une quelconque des revendications précédentes, **caractérisé en ce qu'**un diélectrique au moins (460a, 460b) est réalisé en TMM^{®}.

8. Résonateur à hyperfréquences selon l'une quelconque des revendications précédentes, **caractérisé en ce que** le tube de guidage (460b) se présente sous une forme conique au moins en certains de ses segments.

9. Résonateur à hyperfréquences selon l'une quelconque des revendications précédentes, **caractérisé en ce que** le tube de guidage (460b) comporte un orifice d'entrée (461) élargi, de préférence conique.

10. Résonateur à hyperfréquences selon l'une quelconque des revendications précédentes, **caractérisé en ce qu'**un entonnoir (426) en alignement précis avec l'orifice d'extrémité du tube correspondante est disposé ou peut être disposé dans la zone de l'extrémité du tube de guidage (460b) située sur le côté aval de l'écoulement.

11. Résonateur à hyperfréquences selon la revendication 10, **caractérisé en ce que** le tube de guidage (460b) et l'entonnoir (426) se présentent sous forme monocorps.

12. Résonateur à hyperfréquences selon l'une quelconque des revendications précédentes, **caractérisé en ce que** des tubes de guidage (460b) différents pouvant être disposés en alternative dans le résonateur (30) sont prévus, lesquels présentent essentiellement le même diamètre extérieur et des diamètres intérieurs différents l'un des autres.

13. Résonateur à hyperfréquences selon l'une quelconque des revendications précédentes, **caractérisé en ce que** des tubes de guidage intérieurs (460b) différents avec des diamètres intérieurs différents peuvent être disposés dans le tube extérieur (460a).

14. Résonateur à hyperfréquences selon l'une quelconque des revendications précédentes, **caractérisé en ce que** des tubes de guidage (460b) différents pouvant être disposés en alternative dans le résonateur (30) et avec des diamètres intérieurs différents présentent essentiellement la même masse dans la zone de dispersion des micro-ondes.

15. Machine textile, particulièrement cardeuse, étireuse ou peigneuse, **caractérisée en ce qu'**elle comporte au moins un résonateur à hyperfréquences (30) selon l'une quelconque des revendications précédentes.
